# EUROPEAN PATENT APPLICATION

(11) **EP 4 647 008 A1**
(43) Date of publication of application: **12.11.2025**
(21) Application number: 25175417.2
(22) Date of filing: 09.05.2025
(51) Int. Cl.: A61B 5/287, A61B 5/339, A61B 5/367, A61B 5/00

(54) **CARDIAC MAP ADVANCED RIPPLE MODE WITH GUI**

(30) Priority: 10.05.2024 US 202463645336 P; 27.03.2025 US 202519091925
(71) Applicant: Biosense Webster (Israel) Ltd., 2066717 Yokneam (IL)
(72) Inventor: SEGEV, Meytal, 2066717 Yokneam (IL); BEN NATAN, Alon, 2066717 Yokneam (IL); YANOVICH, Nir, 2066717 Yokneam (IL); KEREN TAL, Dor Zeev, 2066717 Yokneam (IL); ZIDAN, Layla Saleem, 2066717 Yokneam (IL)
(74) Representative: Carpmaels & Ransford LLP

(57) **Abstract**

A system includes an input device and a processor. The processor is configured to (i) one of receive and generate an electrophysiological (EP) map that visualizes a first EP parameter on the EP map with a graphical representation that varies in size according to a value of the first EP parameter, (ii) receive, using the input device, a second EP parameter, (iii) apply a visual indication of the second EP parameter on the graphical representation of the first EP parameter, wherein the visual indication varies according to a value of the second EP parameter, and (iv) display the EP map to the user on a display device.

## Description

### CROSS-REFERENCE TO RELATED APPLICATION

This application claims the benefit of U.S. Provisional Patent Application 63/645,336, filed May 10, 2024, which is incorporated herein by reference.

### FIELD OF THE DISCLOSURE

The present invention relates generally to electrophysiological mapping, and particularly to visualization of cardiac electrophysiological data points and maps.

### BACKGROUND OF THE DISCLOSURE

An electrophysiological (EP) map of a cardiac chamber of a patient is generated by positioning electrodes on a region of the chamber's tissue, acquiring an EP signal of the region, and then repeating the process for a different region. EP parameters are extracted from the EP signals in each region of measurement, and then displayed over a graphical representation of the tissue, such as a three-dimensional (3D) rendering of the cardiac chamber.

EP mapping visualization methods previously proposed in the patent literature, to ease an interpretation of an EP map. For example, U.S. Patent 11,844,616 describes methods, apparatus, and systems for medical procedures that include sensing a plurality of tissue electrical potentials at an organ area of an organ, by one or more electrodes on a catheter. A number of peak electrical potentials is determined from the plurality of first tissue electrical potentials such that the peak electrical potential exceeds a potential threshold. A first visual characteristic is determined based on the number of peak electrical potentials. A rendering of the organ is displayed that comprises the organ area such that the rendering of the first organ area comprises the first visual characteristic.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electrophysiological (EP) mapping and ablation system, in accordance with an example of the present disclosure;
Fig. 2 is a schematic illustration of a graphical user interface (GUI) that lets a user select an EP parameter and configure its binning for display in an EP map, in accordance with an example of the present disclosure;
Fig. 3 is a schematic, pictorial volume rendering of an LAT map of a cardiac chamber superimposed with ripples of fractionation counts of bipolar amplitudes, generated using the GUI of Fig. 2, in accordance with an example of the present disclosure; and
Fig. 4 is a flow chart that schematically illustrates a method for using a GUI and an input device to generate an EP map superimposed with the ripples of fractionation counts seen in Fig. 3, in accordance with an example of the present disclosure.

### DETAILED DESCRIPTION OF EXAMPLES

### OVERVIEW

During an EP mapping procedure, an electrophysiological (EP) map, such as a local activation time (LAT) map, is often generated by an EP mapping system based on EP data captured at a plurality of locations inside a cardiac chamber. The map is a useful tool in the diagnosis of an arrhythmia (e.g., atrial fibrillation) and to direct a physician in eliminating it, e.g., using ablation.

In the procedure, the mapping system applies catheters to sense the activation wave (reference signal) and the resulting activations (substrate signal). A processor annotates the reference signal and the respective substrate activation. Typically, hundreds of waveforms are acquired and annotated during a mapping procedure. Using the annotations, the processor calculates the respective EP value, for example, the local activation time (LAT) value between the reference signal and the respective substrate signal.

In some cases, the processor of the EP mapping system presents discrete EP values of one EP parameter overlaid on a continuous (e.g., interpolated) EP map of another EP parameter. For example, a "ripple map" comprising a dynamic display of discrete bipolar electrogram data can be used in conjunction with a LAT map visualization. As used herein, a "ripple" refers to a bar (or other graphical representation) protruding from a surface of an electroanatomical map, where the size (e.g., height or width) of the bar represents a particular EP value. In the CONFIDENSE^{®} Module with Ripple Mapping available from Biosense Webster, Inc., ripples provide a dynamic display of bipolar electrogram data where, at each point, the corresponding bipolar electrogram amplitude is dynamically displayed as a bar perpendicular to the LAT map surface. A limitation of the existing ripple implementations, such as in the CONFIDENSE^{®} Module, is that the bars overlaid on the anatomical map conveys a measure of only one the EP parameter: the bipolar amplitude at the bar position. The bar representation may indicate an anomalous conduction path causing an arrhythmia. However, by providing only a singular form of EP information, where that information is represented exclusively by the height of the ripple, this kind of visualization may be too coarse or difficult to observe and rely upon for diagnosis.

Examples of the present invention that are described hereinafter provide novel ripple mapping systems and methods that enable ripples to simultaneously convey additional, valuable EP parameter information beyond bipolar amplitude. In an example, a system is provided that includes a display device, an input device (e.g., a touch screen or a computer mouse), and a processor. The processor is configured to receive or generate an electrophysiological (EP) map that visualizes a first EP parameter (e.g., bipolar amplitude) on the EP map with a graphical representation (e.g., bar height) that varies in size according to a value of the first EP parameter. The processor is further configured to receive a second EP parameter (e.g., signal fractionation count), and apply a visual indication (e.g., color) of the second EP parameter on the graphical representation of the first EP parameter, wherein the visual indication varies according to a value of the second EP parameter. The EP map is displayed to the user on the display device or is stored in memory.

The processor may receive the selection of the second EP parameter from the user via the input device. In other examples, the processor further receives from the user via the input device, a binning of a range (e.g., three bins of count groups on a scale ranging between 0 to 10) of the second EP parameter. The processor applies a visual indication (e.g., bar color, transparency, intensity, hue, hatching, etc.) of the second EP parameter upon the given graphical representation of the first EP parameter.

In some examples, the processor is further configured to provide a graphical user interface (GUI) feature that lets a user select the second EP parameter, and configure its binning, using the display device and the input device. The GUI feature lets a user configure a binning of the second EP parameter by the GUI comprising, for example, a slider ruler with two or more user-movable separators of the range of the second EP parameter.

In certain examples, the user can visualize all colors or only a specific color (e.g., selected to represent one or more of high/mid/low binning) of the chosen EP parameter. The color set may be customized. In an example, to graphically represent the binning of a range of the EP parameter (e.g., to give different colors to the ripple bars), the user moves the separators on the slider ruler to visualize a with a deflection count of 0-3 with a first color bar, a count of 3-7 for a second color bar, and a deflection count larger than 7 for a third color bar, as seen below. As noted above, the user may select on the GUI to show only one bin, such as showing the high fractionation count.

In some examples, the technique offers a ripple mode with a graphical representation in the form of colored bars, the size (e.g., height or width) of the geometric representation (e.g., bar) representing bipolar amplitude of a measured signal at the location. Ripples may be modified with visual indications (e.g., color, transparency, intensity, hue, hatching, etc.) that vary according to another EP parameter of interest measured or derived for the location of the ripple, such as, but not limited to, fractionation count, activation duration, late potential level of sensitivity, quality of electrode contact with tissue, and others.

In some examples, the processor may superimpose the graphical representation (i.e., ripples) with the applied visual indication upon an existing EP map, such as an LAT or voltage map on a display for the user.

Finally, the graphical representations can be permutated between applications, e.g., reversed, where in examples where a ripple map is superimposed with a surface EP mapping, the second EP parameter may be a LAT value with bar height as representing the LAT value. Bar color can represent the LAT value and bar height representing the second EP parameter value.

### SYSTEM DESCRIPTION

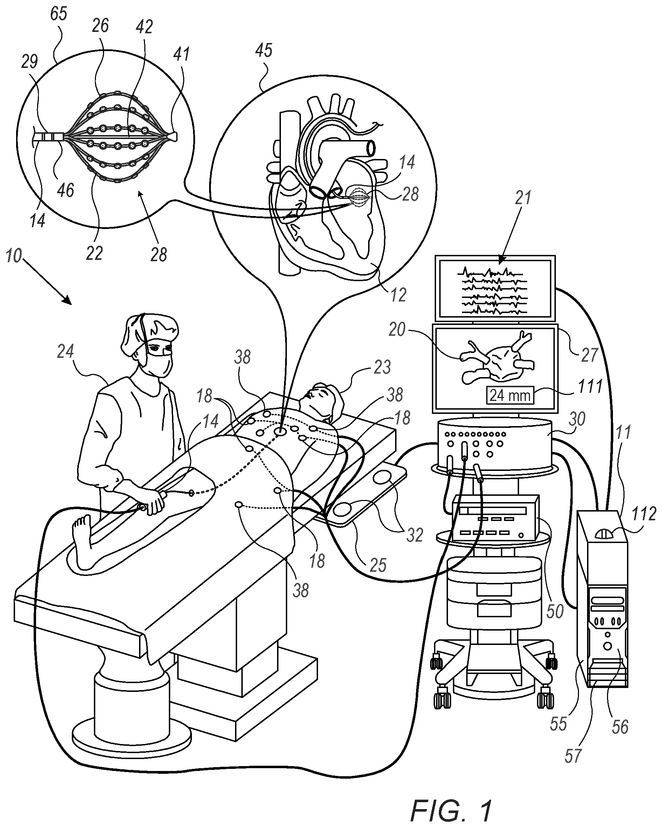

Fig. 1 is a schematic, pictorial illustration of a catheter-based electrophysiological (EP) mapping and ablation system 10, in accordance with an example of the present disclosure.

System 10 includes multiple catheters which are percutaneously inserted by physician 24 through the patient's vascular system into a chamber or vascular structure of a heart 12 (seen in inset 45). Typically, a delivery sheath catheter is inserted into a cardiac chamber, such as a ventricle or an atrium, near a desired location in heart 12. Thereafter, a plurality of catheters is inserted into the delivery sheath catheter to arrive at the desired location. The plurality of catheters may include a catheter dedicated for pacing, a catheter for sensing intracardiac electrogram signals, a catheter dedicated for ablating and/or a catheter dedicated for both EP mapping and ablating. An example catheter 14, illustrated herein, is configured for sensing bipolar electrograms. Physician 24 brings a distal tip 28 (also called hereinafter distal end assembly 28) of catheter 14 into contact with the heart wall for sensing a target site in heart 12. For ablation, physician 24 similarly brings a distal end of an ablation catheter to a target site.

As seen in inset 65, catheter 14 is an exemplary catheter that includes a basket distal end 28, including one, and preferably multiple, electrodes 26 optionally distributed over a plurality of splines 22 at distal tip 28 and configured to sense IEGM signals. Catheter 14 may additionally include a position sensor 29 embedded in or near distal tip 28 on a shaft 46 of catheter 14, which is used to track the position and orientation of distal tip 28. Optionally, and preferably, position sensor 29 is a magnetic-based position sensor including three magnetic coils for sensing three-dimensional (3D) position and orientation. As seen, distal tip 28 further includes an expansion/collapse rod 42 of expandable assembly 28 that is mechanically connected to basket assembly 28 at a distal edge 41 of assembly 28.

Magnetic based position sensor 29 may be operated together with a location pad 25 that includes a plurality of magnetic coils 32 configured to generate magnetic fields in a predefined working volume. Real-time position of distal tip 28 of catheter 14 may be tracked based on magnetic fields generated with location pad 25 and sensed by magnetic based position sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays, on display device 27, cardiac signals 21 (e.g., electrograms acquired at respectively tracked cardiac tissue positions) acquired with body surface ECG electrodes 18 and intracardiac electrograms acquired with electrodes 26 of catheter 14. Recorder 11 may include pacing capability to pace the heart rhythm, and/or may be electrically connected to a standalone pacer.

Workstation 55 includes memory 57, a processor 56 unit with memory or storage with appropriate operating software loaded therein, and user interface capability. Workstation 55 may provide multiple functions, optionally including (i) modeling endocardial anatomy in three-dimensions (3D) and rendering the model or EP map 20 for display on display device 27, (ii) displaying on display device 27 activation sequences (or other data) compiled from recorded cardiac signals 21 in representative visual indicia or imagery superimposed on the rendered EP map 20, (iii) displaying real-time location and orientation of multiple catheters within the heart chamber, and (iv) displaying sites of interest on display device 27, such as places where ablation energy has been applied. One commercial product embodying elements of system 10 is available as the CARTO^{™} 3 System, available from Biosense Webster, Inc., 31A Technology Drive, Irvine, CA 92618.

In a disclosed example, physician 24 uses a GUI 111 that includes a signal type menu to select (e.g., using a computer mouse 112) which EP parameter will be graphically visualized, according to the disclosed technique. An input device 110 (e.g., one comprising a touchscreen 27 or computer mouse 112) is configured to let physician 24 set or adjust the selected EP parameter, and to configure its binning, on GUI 111.

System 10 may include an ablation energy generator 50 that is adapted to conduct ablative energy to one or more electrodes at a distal tip of a catheter configured for ablation. Energy produced by ablation energy generator 50 may include, but is not limited to, radiofrequency (RF) energy or pulsed-field ablation (PFA) energy, including monopolar or bipolar high-voltage DC pulses, to be used to effect irreversible electroporation (IRE), or combinations thereof.

Patient interface unit (PIU) 30 is an interface configured to establish electrical communication between catheters, electrophysiological equipment, power supply and a workstation 55 to control system 10 operation and to receive EA signals from the catheter. Electrophysiological equipment of system 10 may include, for example, multiple catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations.

In some examples, processor 56 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

This configuration of system 10 is shown by way of example, to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other types of medical systems. For example, other multi-electrode catheter types may be used, such as the multi-arm OCTARAY^{™} catheter or a flat catheter.

### GUI FOR EP MAP WITH ADVANCED RIPPLE MODE

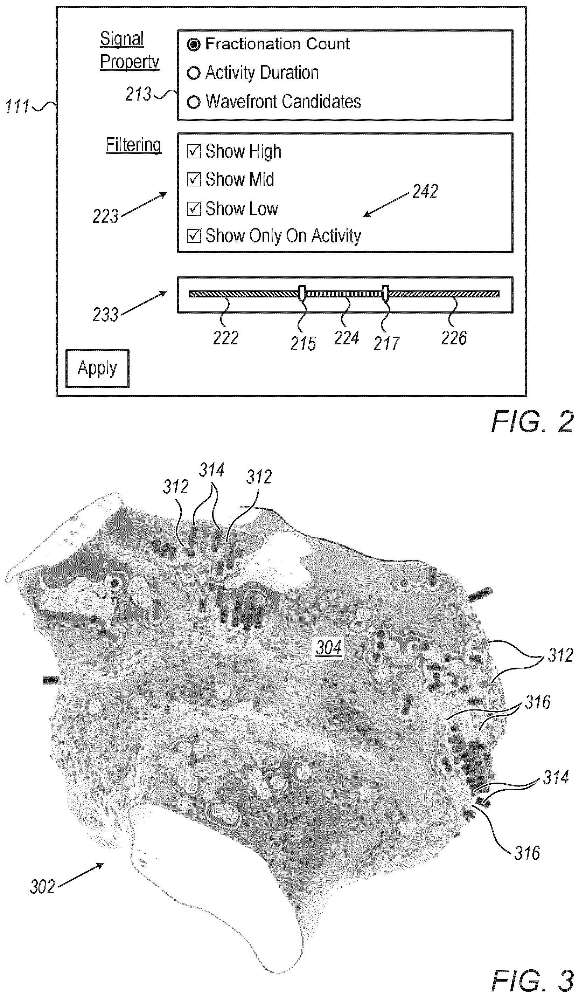

Fig. 2 is a schematic illustration of GUI 111 that lets a user select an EP parameter and configure its binning over the range of the EP parameter for display in an EP map, in accordance with an example of the present disclosure.

In the shown example, the ripple bars have different heights according to their bipolar value (amplitude) representing the first visualized EP parameter. GUI 111 is configured to allow a user to select, using checkboxes, a second EP parameter, and to configure its binning to be graphically represented as bar colors.

In the shown example, GUI 111 comprises a signal property menu 213 that allows the user to select a graphical visualization of a second EP parameter from among fractionation count, activity duration, and wavefront.

GUI 111 further comprises a filtering menu 223 that allows a user to configure a sliding ruler scale 233. As seen, menu 223 allows a user to configure the visualization of the second EP parameter with up to three binning levels (low, mid, high). For example, the user may select only the high check box, which will show only areas with high fractionation counts (e.g., counts 316 colored red in Fig. 3).

Menu 223 further includes a checkbox 242 to show bars on activity areas only, or all over the map. Activity areas are defined elsewhere.

As shown, GUI 111 further comprises the configured sliding ruler scale 233. Ruler scale 233 comprises low (222), mid (224) and high (226) binning, colored respectively in green, yellow, and red. The user may configure the binning ranges using sliding separators 215 and 217.

### EP MAP DISPLAYING ADVANCED RIPPLE MODE

Fig. 3 is a schematic, pictorial volume rendering of an LAT map 302 of a cardiac chamber superimposed with ripples of fractionation counts (222, 224, 226) of bipolar amplitudes, in accordance with an example of the present disclosure. The map is generated using GUI 111 of Fig. 2. In LAT map 302, the continuously represented first EP parameter 304 (e.g., LAT values 304) is coded by a color scale. The first EP parameter 324 of different bipolar amplitudes is graphically represented by bars 310 of different heights 320.

The different bar 310 colors (312, 314, 316) represent the second EP parameter 334 of the level of fractionation according to the binning set by the user. In the shown example, the binning is set using ruler separators 215 and 217 to show a low count of up to three deflections 223 (green, 312), between 3-6 deflections count 224 (yellow, 224), and above six deflections count 226 (red, 316). Depending on the selection in signal selection menu 213, the algorithm can look at, and set levels for, duration or additional inputs other than fractionation count.

LAT map 302 can characterize atrial flutter (AF) with areas of activity with high deflection count (e.g., as selected in ruler 233 to be greater than 6) and a range of bipolar values of 0.05-0.5 mV. LAT map 302 can characterize atrial fibrillation (Afib) where areas of activity have a high deflection count (e.g., as selected in ruler 233 to be greater than 6) and a range of bipolar values of 0.05-2 mV.

### METHODS TO GENERATE EP MAP WITH ADVANCED RIPPLE MODE

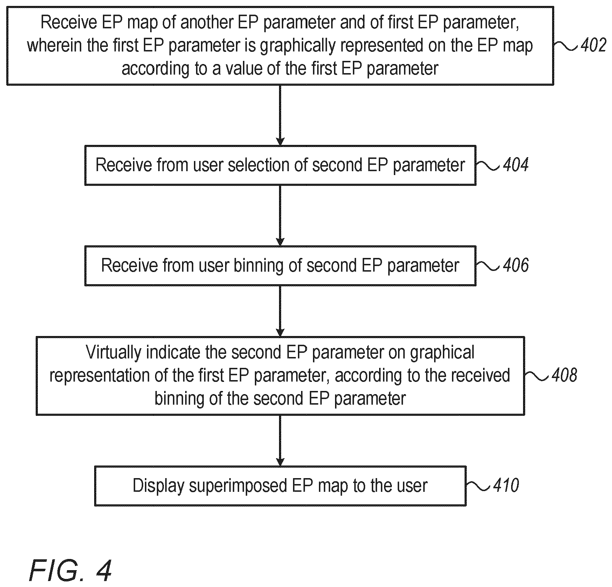

Fig. 4 is a flow chart that schematically illustrates an example method for using GUI 111 and input device 110 to generate an EP map 302 superimposed with the ripples of fractionation counts (312, 314, 316) seen in Fig. 3, in accordance with an example of the present disclosure.

The process carries out an algorithm that begins with processor 56 receiving an EP map of certain another EP parameter (e.g., LAT) and of a first EP parameter (e.g., bipolar amplitude), wherein the first EP parameter is graphically represented on the EP map with a given graphical representation (e.g., bars of different heights) according to a value of the first EP parameter, at EP map receiving step 402.

At a second EP parameter receiving step 404, the processor receives from the user, via input device 110, a selection on GUI 111 of a second EP parameter (e.g., signal fractionation count).

At a binning receiving step 404, the processor receives a configuration done on GUI 111 of a binning of a range of the second EP parameter from the user via input device 110.

At visual indication step 408, the processor visually indicates (e.g., colors each bar) the second EP parameter on the graphical representation (e.g., the bars) of the first EP parameter according to the received binning of the second EP parameter.

At EP map displaying step 410, the processor displays the EP map (e.g., map 302) to the user.

### EXAMPLES

### Example 1

A system (10) includes an input device (110) and a processor (56). The processor (56) is configured to (i) one of (i) one of receive and generate an electrophysiological (EP) map (302) that visualizes a first EP parameter (324) on the EP map (302) with a graphical representation (310) that varies in size (320) according to a value of the first EP parameter, (ii) receive, using the input device (110), a second EP parameter (334), (iii) apply a visual indication (312, 314, 316) of the second EP parameter (334) on the graphical representation (310) of the first EP parameter (324), wherein the visual indication (312, 314, 316) varies according to a value of the second EP parameter (334), and (iv) display the EP map (302) to the user.

### Example 2

The system (10) according to example 1, wherein the processor (56) is further configured to receive from a user, via the input device (110), a binning (222, 224, 226) of a range of the second EP parameter (334) and apply the visual indication (312, 314, 316) of the second EP parameter (334) according to the received binning (222, 224, 226).

### Example 3

The system (10) according to any of examples 1 and 2, wherein the processor (56) is configured to provide, using a display device (27) and the input device (110), a graphical user interface (GUI) (111) feature that lets a user to at least one of: i) select the second EP parameter (334) and ii) configure its binning.

### Example 4

The system (10) according to any of examples 1 through 3, wherein the GUI feature (111) lets the user configure the received binning (222, 224, 226) using a slider ruler (233) with one or more user-movable separators (215, 217) of the range of the second EP parameter (334).

### Example 5

The system (10) according to any of examples 1 through 4, wherein the first EP parameter (324) is bipolar electrocardiogram amplitude.

### Example 6

The system (10) according to any of examples 1 through 5, wherein the second EP parameter (334) is one of fractionation count, activity duration, and wavefront candidates.

### Example 7

The system (10) according to any of examples 1 through 6, wherein the processor (56) is configured to configure the visual indication (320) of the first EP parameter as bars (310) having heights (320) according to the first EP parameter (324) value.

### Example 8

The system (10) according to any of examples 1 through 7, wherein the processor (56) is configured to color (312, 314, 316) the bars (310) according to the received binning (222, 224, 226).

### Example 9

The system (10) according to any of examples 1 through 8, wherein the processor (56) wherein the processor is configured to visualize the first EP parameter (324) on an EP map of another EP parameter (304).

### Example 10

The system (10) according to any of examples 1 through 9, wherein the other EP parameter (304) is local activation time (LAT)

### Example 11

The system (10) according to any of examples 1 through 10, wherein the input device (110) comprises one of a touchscreen (27) and a computer mouse (112).

### Example 12

A method includes one of receiving and generating an electrophysiological (EP) map (302) that visualizes a first EP parameter (324) on the EP map (302) with a graphical representation (310) that varies in size (320) according to a value of the first EP parameter (324). A second EP parameter (334) is received. A visual indication (312, 314, 316) of the second EP parameter (334) is applied on the graphical representation (310) of the first EP parameter (324), wherein the visual indication (312, 314, 316) varies according to a value of the second EP parameter (334). The EP map (302) is displayed to a user.

### Example 13

The method according to example 12, and comprising receiving from the user a binning of a range of the second EP parameter and applying the visual indication of the second EP parameter according to the received binning.

### Example 14

The method according to example 13, and comprising providing a graphical user interface (GUI) feature that lets the user to at least one of: i) select the second EP parameter and ii) configure its binning.

### Example 15

The method according to example 14, wherein the GUI feature lets a user configure the received binning using a slider ruler with one or more user-movable separators of the range of the second EP parameter.

### Example 16

The method according to example 12, wherein the first EP parameter is bipolar electrocardiogram amplitude.

### Example 17

The method according to example 12, wherein the second EP parameter is one of fractionation count, activity duration, and wavefront candidates.

### Example 18

The method according to example 12, and comprising configuring the visual indication of the first EP parameter as bars having heights according to the first EP parameter value.

### Example 19

The method according to example 18, and comprising coloring the bars according to the received binning of the second EP parameter.

### Example 20

The method according to example 12, wherein the processor is configured to visualize the first EP parameter on an EP map of another EP parameter.

### Example 21

The method according to example 20, wherein the another EP parameter is local activation time (LAT).

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A system, comprising:
an input device (110); and
a processor (56), configured to:
one of receive and generate an electrophysiological (EP) map (302) that visualizes a first EP parameter (324) on the EP map (302) with a graphical representation (310) that varies in size (320) according to a value of the first EP parameter (324);
receive, using the input device (110), a second EP parameter (334);
apply a visual indication (312, 314, 316) of the second EP parameter (334) on the graphical representation (310) of the first EP parameter (324), wherein the visual indication (312, 314, 316) varies according to a value of the second EP parameter (334); and
display the EP map (302) to a user.

2. The system according to claim 1, wherein the processor (56) is further configured to receive from the user, via the input device (110), a binning (222, 224, 226) of a range of the second EP parameter (334) and apply the visual indication (312, 314, 316) of the second EP parameter (334) according to the received binning (222, 224, 226).

3. The system according to claim 2, wherein the processor (56) is configured to provide, using a display device (27) and the input device (110), a graphical user interface (GUI) feature (111) that lets the user to at least one of: i) select the second EP parameter (334) and ii) configure its binning.

4. The system according to claim 3, wherein the GUI feature (111) lets a user configure the received binning (222, 224, 226) using a slider ruler (233) with one or more user-movable separators (215, 217) of the range of the second EP parameter (334).

5. The system according to any one of claims 1 to 4, wherein the first EP parameter (324) is bipolar electrocardiogram amplitude.

6. The system according to any one of claims 1 to 5, wherein the second EP parameter (334) is one of fractionation count, activity duration, and wavefront candidates.

7. The system according to any one of claims 1 to 6, wherein the processor (56) is configured to configure the visual indication of the first EP parameter (324) as bars (310) having heights (320) according to the first EP parameter (324) value.

8. The system according to claim 7, wherein the processor (56) is configured to color (312, 314, 316) the bars (310) according to the received binning (222, 224, 226) of the second EP parameter (334).

9. The system according to any one of claims 1 to 8, wherein the processor (56) is configured to visualize the first EP parameter (324) on an EP map of another EP parameter (304).

10. The system according to claim 9, wherein the other EP parameter (304) is local activation time (LAT).

11. The system according to any one of claims 1 to 10, wherein the input device (110) comprises one of a touchscreen (27) and a computer mouse (112).

12. A method, comprising:
receiving an electrophysiological (EP) map (302) that visualizes a first EP parameter (324) on the EP map (302) with a graphical representation (310) that varies in size (320) according to a value of the first EP parameter (324);
receiving a second EP parameter (334);
applying a visual indication (312, 314, 316) of the second EP parameter (334) on the graphical representation (310) of the first EP parameter (324), wherein the visual indication (312, 314, 316) varies according to a value of the second EP parameter (334); and
displaying the EP map (302) to a user.

13. The method according to claim 12, and comprising receiving from the user a binning (222, 224, 226) of a range of the second EP parameter (334) and applying the visual indication (312, 314, 316) of the second EP parameter (334) according to the received binning (222, 224, 226).

14. The method according to claim 13, and comprising providing a graphical user interface (GUI) feature (111) that lets the user to at least one of: i) select the second EP parameter (334) and ii) configure its binning.

15. The method according to claim 14, wherein the GUI feature (111) lets a user configure the received binning (222, 224, 226) using a slider ruler (233) with one or more user-movable separators (215, 217) of the range of the second EP parameter (334).
